# EUROPÄISCHE PATENTANMELDUNG

(11) **EP 2 399 534 A1**
(43) Veröffentlichungstag der Anmeldung: **28.12.2011**
(21) Anmeldenummer: 10167019.8
(22) Anmeldetag: 23.06.2010
(51) Int. Cl.: A61B 17/92, A61B 17/16

(54) **Extraktionswerkzeug für das Entfernen von Knochenschrauben, insbesondere in der Orthopädie oder der Unfallchirurgie**

(71) Anmelder: PB Swiss Tools GmbH, 3457 Wasen im Emmental (CH)
(72) Erfinder: Aeschlimann, Kurt, 3457, Wasen (CH); Schüpbach, Peter, 3457, Wasen (CH)
(74) Vertreter: Liebetanz, Michael

(57) **Zusammenfassung**

Ein Extraktionswerkzeug für das Entfernen von Knochenschrauben, insbesondere in der Orthopädie oder der Unfallchirurgie, umfasst einen Hohlfräser (12), der über das zu extrahierende Werkstück (51) einsetzbar ist, um einen dieses umgebenden Kanal (53) zu bohren. Dabei verfügt der Hohlfäser (12) an seinem proximalen Ende über spanabhebende Elemente und in seinem hohlen, zentrischen Innenraum über Eingriffselemente, so dass das Werkzeug nach einer vorbestimmbaren Vordringtiefe mit dem zu extrahierenden Werkstück (51) so fest verbunden ist, dass dieses extrahierbar ist. Dabei verfügt die hohle Innenseite (21, 22) des Werkzeuges über einen mindestens teilweise glatten, in seinem Durchmesser sich distal verjüngenden Innenwandabschnitt (22).

## Beschreibung

### TECHNISCHES GEBIET

Die Erfindung betrifft ein Extraktionswerkzeug für das Entfernen von Knochenschrauben, insbesondere in der Orthopädie oder der Unfallchirurgie, mit den Merkmalen gemäss dem Oberbegriff des Anspruchs 1.

Ferner betrifft die Erfindung Einwegwerkzeuge für die Chirurgie mit einem Kennzeichen zur Sicherstellung ihres einmaligen Einsatzes.

### STAND DER TECHNIK

Werkzeuge zur Entfernung von in einem Material eingelassenen Werkstücken, insbesondere wenn das Werkstück keinen Betätigungskopf mehr aufweist, sind seit langem bekannt. Hierzu gehören auch Extraktionswerkzeuge für das Entfernen von Knochenschrauben, insbesondere in der Orthopädie und Unfallchirurgie des Menschen oder des Tiers. Dabei ist das Material Knochen, das Werkstück eine zumeist mit einem Gewinde in dem Knochen eingedrehte Schraube, bei der zum Beispiel der Schraubenkopf beschädigt ist oder die als ganzes so nicht aus dem Knochen herausgedreht werden kann.

Ein Werkzeug mit den Merkmalen des Oberbegriffs des Anspruchs 1 ist aus der US 1,548,835 bekannt, bei der eine Hohlfräse über das zu extrahierende Werkstück eingesetzt wird und einen Kanal bohrt. Die hohle Innenseite des Werkzeuges ist mit einem Innengewinde versehen, welches selbstschneidend das zu extrahierende Werkstück bearbeitet und nach einer gewissen Vordringtiefe mit diesem so fest verbunden ist, dass die Schraube in entgegensetzter Eindrehrichtung entfernt werden kann.

Dieser Extraktor erfordert einen gewisse Höhe des Schraubenkopfs als überstehender Bereich und eine entsprechend angepasste konische Innenform, die das selbstschneidende Gewinde trägt. Dies erfordert dadurch das Bereithalten einer Vielzahl von solchen Extraktionswerkzeugen für unterschiedliche Schraubendurchmesser.

In den nachfolgenden Jahren hat es eine Reihe von Weiterentwicklungen gegeben. Die US 6,267,025 beschreibt eine Hohlfräse als Extraktionswerkzeug mit einem nach hinten geführten Ablaufkanal für abgehobene Späne; ansonsten arbeitet der Extraktor nach der US 6,267,025 wie sein 75 Jahre älteres Pendant und schneidet ein Gewinde in die zu entfernende Knochenschraube.

Aus der DE 20 2004 013 291 ist ein Werkzeug zum Lösen und Festziehen eines Werkstückes bekannt, welches eine Hülse umfasst, bei der innen eine konische Verjüngung mit nicht verzahnten und verzahnten Spiralflächen abwechselnd besetzt ist, wobei je nach Bedarf die nicht verzahnten oder die verzahnten Spiralflächen zum Drehen des Werkstücks verwendet werden können. Das Werkzeug ist nur einsetzbar, wenn der entsprechende ringförmige Hohlraum durch einen entsprechenden Hohlbohrer vorbereitet ist.

Die US 6,877,402 betrifft ein weiteres Extraktionswerkzeug mit einer Hülse, deren innen hohler Raum bogenförmig verlaufende Nuten aufweist, welche sich verjüngend in das Innere des Extraktionswerkzeuges fortsetzen. Auch hier ist kein dem Extraktionswerkzeug zugeordneter Bohrer offenbart.

Chirurgen verwenden häufig zuerst eine Hohlfräse gemäss dem Stand der Technik, um einen Hohlraum um das beschädigte Werkstück zu erzeugen, wonach nachfolgend ein Extraktor in einem zweiten Schritt ähnlich zur US 6,877,402 oder DE 20 2004 013 291 in den aufgebohrten Freiraum vorgeschoben wird und nach Schneiden eines Gewindes die dann festgesetzte Schraube in Gegenrichtung aus dem Knochenmaterial herausgedreht wird.

### DARSTELLUNG DER ERFINDUNG

Der Stand der Technik verlangt zum einen relativ weit über das Knochenmaterial überstehende Stumpfreste der Werkstücke und greift zudem weit und tief um das zu extrahierende Werkzeug ein. Andererseits schlägt er immer ein zweistufiges Verfahren vor, bestehend aus Erzeugen eines Hohlraums um die zu extrahierende Schraube und anschliessendes Schneiden eines (neuen) Gewindes auf der zu extrahierenden Schraube, um dann diese in einer Gegendrehung zu entfernen. Dabei besteht beim Fräsen und dem nachfolgenden Ersatz des Hohlfräsers zum Einsatz des Extraktionswerkzeugs einerseits immer das Risiko des Verlustes von Spanmaterial, insbesondere im menschlichen Körper, und andererseits bedeutet die Zweistufigkeit eine zeitliche Verlängerung des Extraktionsschrittes. Diese Zweistufigkeit wird von verschiedenen Chirurgen daher aus beiden Gründen als sehr störend angesehen.

Ausgehend von diesem Stand der Technik liegt der Erfindung die Aufgabe zugrunde, ein verbessertes Extraktionswerkzeug anzugeben, insbesondere eines, welches schneller und einfacher handhabbar ist.

Ferner ist es ein Ziel der vorliegenden Erfindung die Auswahl des passendes Extraktionswerkzeuges für den Chirurgen zu vereinfachen.

Schliesslich ist es ein weiteres Ziel, die bei der Fräsarbeit entstehende Spanmenge möglichst zu vermindern und deren Verbleib im Körper zu verhindern.

Bei Hohlfräsern nach dem Stand der Technik werden diese zum Freibohren von abgebrochenen Schrauben vorgesehen, damit diese durch die so entstandene Ansenkung um den Schraubenschaft mit einem geeigneten Werkzeug ergriffen und entfernt werden können. Dabei bleibt ein Teil des ausgebohrten Knochens im zentrischen hohlen Innenraum des Hohlfräsers stecken, der bei einer Sterilisation nicht immer entfernt wird und er kann bei nachfolgender Operation mit dem anscheinend sterilen Instrument zu Kontaminierung führen.

Ein weiteres Ziel ist, ein Einwegwerkzeug anzugeben, bei dem das Spitalpersonal nach der Benutzung des Werkzeugs direkt erkennen kann, dass dieses Einwegwerkzeug nicht mehr verwendet werden sollte. Häufig werden Teile des Operationsbesteck dann trotzdem in den Kreislauf der Sterilisation gegeben. Gegebenenfalls kann dann die bereits erfolgte Benutzung hier auch nach einem fälschlicherweise ausgeführten Sterilisationsschritt leicht festgestellt werden.

Die obenstehenden Aufgaben und Ziele der Erfindung werden mit einem Werkzeug der eingangs genannten Art mit den kennzeichnenden Merkmalen des Anspruchs 1 gelöst, wobei die hohle Innenseite des Werkzeuges über einen mindestens teilweise glatten, in seinem Durchmesser sich distal verjüngenden Innenwandabschnitt verfügt, so dass das Werkzeug nach einer vorbestimmbaren Vordringtiefe mit dem zu extrahierenden Werkstück so fest verbunden ist, dass dieses extrahierbar ist.

Dadurch besteht bei der Schraubenextraktion kaum Abrieb, da die Schraube durch den Bohrdruck im sich für sie verjüngenden Konus verkeilt.

Weitere vorteilhafte Ausführungsbeispiele der Erfindung sind in den Unteransprüchen gekennzeichnet.

Die Erfindung beruht auf der Einsicht, dass das beim Fräsen entstehende Knochenmaterial zwischen der Fräse und dem beschädigten Werkstück komprimierbar ist und damit beim Schritt des Fräsens gleichzeitig einen mit dem zu extrahierenden Werkstück verbundenen Pfropfen bildet. Dieser gestattet eine direkte Extraktion der Schraube. Dabei wird zudem das Knochenmaterial mit der extrahierten Schraube mit entfernt, ohne das Knochenspäne oder Schraubenkopfmaterial in dem Fräskanal verbleiben können.

Gegebenenfalls kann der Operateur mit dem gerade im Einsatz befindlichen Extraktionswerkzeug noch eine zweite zu entfernende Knochenschraube bearbeiten, da das zusammengepresste Knochenmaterial, wenn es nicht bei der Entfernung der Schraube aus dem Werkzeug herausfällt, durch den erneuten Einsatz weiterverdichtet wird.

Das weitere Ziel des Feststellen des erfolgten Einsatzes eines solchen Werkzeuges und somit das sichere optische Vermeiden des erneuten Einsatzes nach einem Sterilisationsschritt wird dadurch gelöst, dass das Werkzeug über einen Kernkörper aus einem Stahl vorbestimmter Härte besteht und dass das Werkzeug mindestens im Bereich der Werkzeugenden eine Beschichtung aufweist, die aus einer kurzzeitig korrosionsbeständigen Plasmabeschichtung besteht. Die Plasmabeschichtung kann eine grössere Härte als der Stahl aufweisen.

Durch die Reinigung eines Werkzeuges, nicht notwendigerweise aber auch eines Fräswerkzeuges, nach dem Einsatz im Umfeld eines menschlichen Knochens oder eines tierischen Knochens ergibt sich eine Umwandlung der erfindungsgemässen Beschichtung des Werkzeuges, so dass die entsprechend irisierende Oberfläche optisch stark verändert aussieht. Hierbei ist es von Vorteil, dass das eigentlich sehr harte Fräswerkzeugmaterial ein sehr günstiger Stahl wie ein HSS Stahl oder ein Federstahl sein kann, insbesondere also kein rostfreies Material, welches durch die Beschichtung einen temporären Korrosionsschutz erfährt. Somit ist ein kostengünstiges Einwegwerkzeug angegeben, neben einem Extraktionswerkzeug auch ein Schraubendreher, Bohrer etc..

Dabei können Instrumente gemäss der Erfindung bevorzugt in Instrumentensortimenten eingesetzt werden. Dabei ist nachteilig, wenn wiederbenutzbare Instrumente unsteril werden, weil aus Kostengründen meist kein zweites System verfügbar ist.

Vorteilhafterweise bestehen solche Wechseleinsätze aus nicht korrosionsbeständigem, gehärtetem Federstahl, welcher durch die, insbesondere härtere, Plasmabeschichtung die verbesserten Oberflächeneigenschaften wie Sichtbarkeit einzelner Sortimentbestandteile für den einmaligen Gebrauch erhält. Federstahl oder HSS-Stahl kann vorgängig zur Plasmabeschichtung bekannten Härteverfahren ausgesetzt werden, was eine höhere Elastizität und Zähigkeit für den einmaligen Einsatz erreicht.

Die härtere Plasmabeschichtung liefert eine temporär korrosionsbeständige Schutzschicht, die durch die über sie mögliche Farbgebung der Oberfläche gleichzeitig als Grössenkodierung einsetzbar ist.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine perspektivische Ansicht eines Werkzeuges gemäss einem Ausführungsbeispiel der Erfindung;
- Fig. 2: eine Seitenansicht der Bearbeitungsspitze des Werkzeuges nach Fig. 1;
- Fig. 3: einen Querschnitt durch die Bearbeitungsspitze des Werkzeuges nach Fig. 2;
- Fig. 4: einen Querschnitt durch die Bearbeitungsspitze eines Werkzeuges gemäss einem zweiten Ausführungsbeispiel nach Fig. 1;
- Fig. 5: einen Querschnitt durch die Bearbeitungsspitze eines Werkzeuges gemäss einem dritten Ausführungsbeispiel nach Fig. 1;
- Fig. 6: einen Querschnitt durch die Bearbeitungsspitze eines Werkzeuges gemäss einem vierten Ausführungsbeispiel nach Fig. 1;
- Fig. 7: ein schematischer Seitenquerschnitt durch die Bearbeitungsspitze des Werkzeuges nach Fig. 1 mit zu entfernendem Schraubenschaft und umgebendes Material; und
- Fig. 8: eine perspektivische Ansicht von sechs Werkzeugspitzen in einer Übersicht.

### BESCHREIBUNG BEVORZUGTER AUSFÜHRUNGSFORMEN

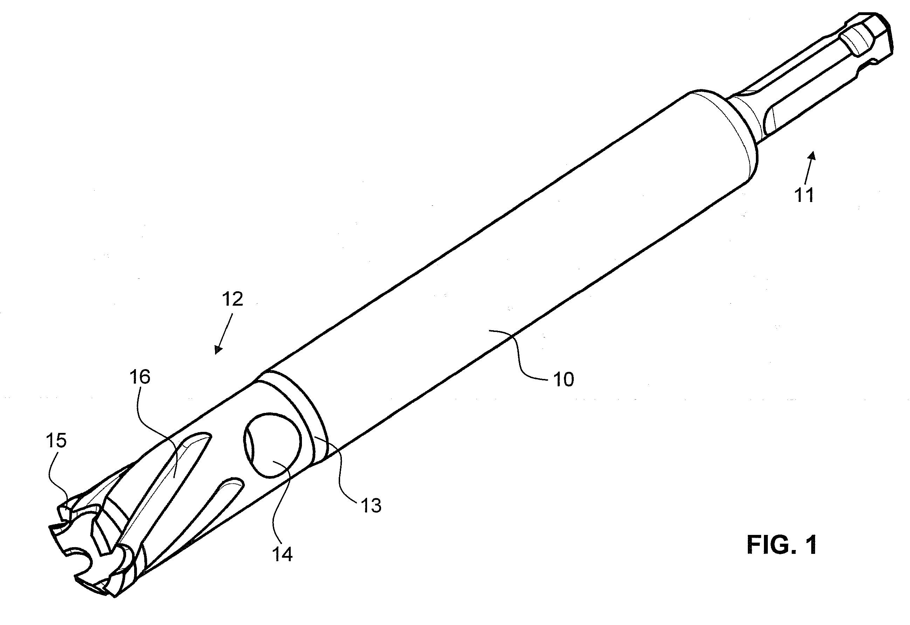

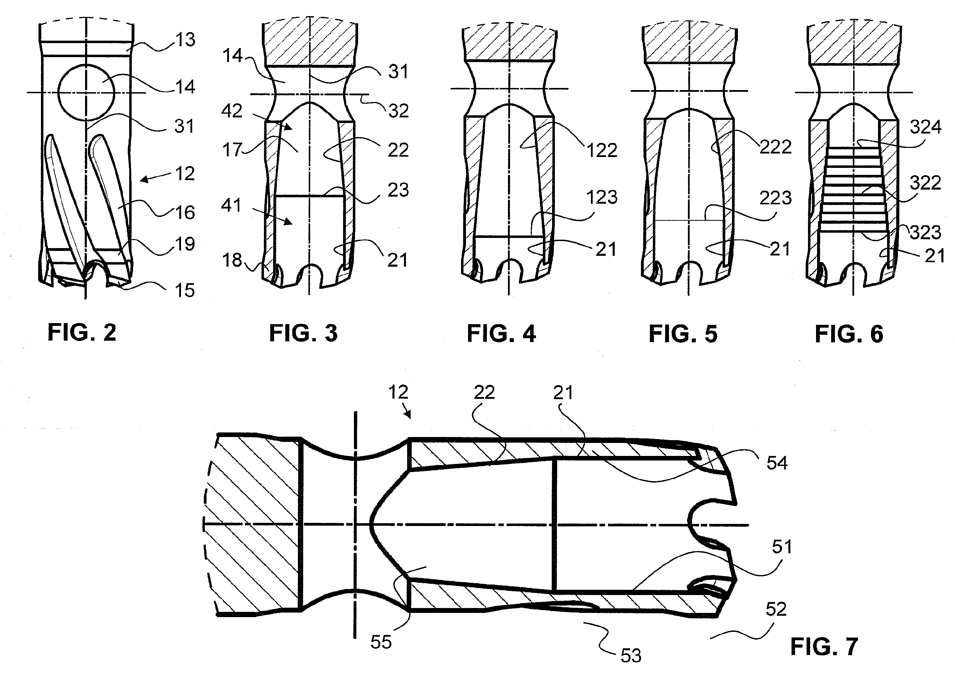

Die Fig. 1 zeigt eine perspektivische Ansicht eines Extraktionswerkzeuges gemäss einem Ausführungsbeispiel der Erfindung. Das Werkzeug besteht aus einem Schaft 10, an dem sich distal ein Verbindungsadapter 11 zur Befestigung in einer Bohrmaschine befindet und wo an dem gegenüberliegenden proximalen Ende der Werkzeugkopf 12 angeordnet ist. Der Werkzeugkopf 12 ist eine Hohlfräse, wie sie in der genaueren Darstellung der Figs. 2 und 3 erläutert wird. Der Schaft des Bearbeitungskopfes weist einen geringeren Durchmesser als der Schaft 10 des Werkzeuges auf, wobei ein Schaftübergang 13 das Ende des Bearbeitungskopfes markiert. Quer durch den Bearbeitungskopf 12 ist im vorliegenden Ausführungsbeispiel die optionale Bohrung 14 hindurch gebracht, durch welche überschüssiges Spannmaterial insbesondere in komprimierter Form herausgeführt werden kann. Das Querloch 14 kann in anderen Ausführungsbeispielen auch nur einseitig oder schräg vorgesehen sein.

Die Fig. 2 zeigt eine Seitenansicht der Bearbeitungsspitze des Werkzeugs nach Fig. 1. Gleiche Bezugszeichen bezeichnen gleiche oder ähnliche Merkmale. Der Werkzeugkopf 12 endet in einer Reihe von Fräszähnen 15, die in der Fräsebene betrachtet einen grösseren Durchmesser als der Schaft des Werkzeugkopfes aufweisen, sodass hier ein Schaftübergang 19 vorgesehen ist. Die verdickten Fräszähne 15 gestatten ein leichteres Vorschieben des gesamten Werkzeugkopfes in den durch die Hohlfräse geschaffenen Ring. Ferner sind in der Fig. 2 mehrere Spannabführnuten 16 zu sehen. Die Spannabführnuten 16 enden unterhalb des optional vorgesehenen Querlochs 14.

Die Fig. 3 zeigt eine Querschnittsansicht durch eine Ebene, die durch die longitudinale Achse oder Längsachse 31 des Werkzeuges und die Querlochachse 32 aufgespannt wird. Das Innere der Bearbeitungshohlfräse umfasst einen Hohlraum 17, der von der Spitze 18 an seiner Mündung bis in das spanabführende Querloch 14 führt. In den Figs. 2 und 3 ist gut zu erkennen, dass die Frässpitze 18 verdickt ist und in einem Schaftübergang 19 in einem dünneren Schaft des Fräskopfes der Bearbeitungsspitze 12 übergeht. Auf der Innenseite des Hohlraums 17 bestehen zwei unterschiedliche Hohlraumabschnitte 41 und 42. Der proximale Hohlraumabschnitt 41 weist eine glatte zylindrische Oberfläche auf, sodass er einem Zylindermantel entspricht. Dieser Abschnitt 41 geht in der Höhe des Abschnittsüberganges 23 in den konischen Hohlraumabschnitt 42 über. Hier ist die Innenwand 22 ein sich zum distalen Ende hin verjüngender Innenwandabschnitt. Im Ausführungsbeispiel der Fig. 3 beträgt die Teilung zwischen zylindrischem und konischen Hohlraumabschnitt 41 und 42 ungefähr die Hälfte der Gesamthöhe des Hohlraumes 17. Die Höhe des Hohlraumabschnittes 41 und 42 beträgt ungefähr zwischen dem 1- und 2-fachen des Nenndurchmessers des Abschnitts 41. Der Winkel des sich verjüngenden Hohlraumabschnittes gegenüber der Zylinderwand 21 beträgt zwischen 1 und 10 Grad, vorteilhafterweise zwischen 2 und 7 Grad, vorzugsweise zwischen 3 und 6 Grad.

Aus den Figs. 4 bis 6 gehen weitere Ausführungsbeispiele hervor, die im Anschluss zur Erläuterung der Funktionsweise im Zusammenhang mit Fig. 7 erläutert werden.

Die Fig. 7 zeigt einen schematischen Seitenquerschnitt durch die Bearbeitungsspitze des Werkzeuges nach Fig. 1 mit einem zu entfernendem Schraubenschaft 51 in umgebendem Knochenmaterial 52. Der Schraubenschaft 51 weist beispielsweise eine Nenngrösse von 3, 3,5 oder 4 mm auf. Dazu passend wird eine Hohlfräse mit Bearbeitungsspitze 12 ausgewählt, die einem Zylinderabschnittdurchmesser 21 von beispielsweise 4,5 mm aufweist. Das Verhältnis der Abschnitte 41 zu 42 beträgt wie bei dem Ausführungsbeispiel der Fig. 3 1:1. Es kann beispielsweise zwischen 1:4 und 2:1 variieren. Die Bearbeitungsspitze 12 fräst ein Bohrloch 53 in das Knochenmaterial 52, wobei ein gewisser Knochenmaterialanteil - schraffiert gezeichnet - mit dem Bezugszeichen 54 zwischen Schaft 51 und der Bearbeitungsspitze 12 verbleibt. Durch die konische Form des Abschnitts 22 wird das Knochenmaterial 54 bei zunehmendem Vortrieb der Bearbeitungsspitze 12 im Bereich des Endes des Knochenschraubenschaftes 51 stark komprimiert, was hier durch das Bezugszeichen 55 angedeutet ist. Durch diese Komprimierung im konischen Bereich zwischen Schraubenschaftwand 51 und konischer Innenwand 22 verspannt sich in überraschend effektiver Weise der Schaft in der Bearbeitungsspitze 12 in einer Art, dass durch ein Ziehen an der Bearbeitungsspitze 12 der noch teilweise verankerte Schraubenschaft 51 aus dem Knochenmaterial 52 herausgelöst werden kann. Dabei ist von Vorteil, dass der grosse Anteil der aufgebohrten Knochenmaterialien durch die Verdichtung im Bereich 55 an Ort und Stelle behalten wird und nicht durch einen Verlust die Wunde kontaminieren kann. Hierfür kann es sinnvoll sein, auf das Querloch 14 zu verzichten oder dieses kleiner oder schräg nach hinten herausführend auszugestalten.

Die Fig. 4 zeigt ein zweites Ausfühnmgsbeispiel einer Bearbeitungsspitze 12 der Erfindung. Hier besteht nur ein sehr kurzer zylindrischer Innenwandabschnitt 21, sodass der Schaftübergang 123 im Bereich von 20 zu 80 (oder 1:4) im Bezug auf die longitudinale Grösse der Bearbeitungsspitze 12 verläuft. Bei einem Winkel des konischen Abschnitts 42 zur Längsachse 31 zwischen 1 und 10 Grad ist der konische Innenwandabschnitt 122 durch die grosse Länge geeignet, eine grössere Komprimierung selbst bei eher unterschiedlichem Durchmesser von Knochenschraubenschaft 51 und Nominalinnenwanddurchmesser 21 des Werkzeuges zu erhalten.

Die Fig. 5 zeigt ein weiteres Ausführungsbeispiel, bei dem sich an den zylindrischen Innenwandabschnitt 21 ab dem ungefähr 1/3 höher angeordneten Schaftübergang 223 ein gekrümmter Innenwandabschnitt 222 anschliesst. Gekrümmt bedeutet hier, dass ohne einen tatsächlich erkennbaren Übergang 223 sich der besagte Winkel gegenüber der Längsachse 31 gegenüber dem Innenwandabschnitt 21 langsam vergrössert, beispielsweise von den 0 Grad (der Parallelität) des zylindrischen Innenwandabschnittes auf 10 Grad. Diese Krümmung kann kontinuierlich ansteigen oder in Abschnitten.

In einem in den Figs. nicht dargestellten Ausführungsbeispiel kann auch auf den zylindrischen Innenwandabschnitt 21 vollständig verzichtet werden und ein sich in der Krümmung verstärkender Innenwandabschnitt 222 vorgesehen sein, dessen Steigerung zu Beginn sehr langsam von 0 bis 2 oder 3 Grad ansteigt und sich dann auf eine Steigung von 5 bis 7 Grad gegenüber der longitudinalen Achse 31 der Werkzeugspitze verstärkt. Es ist vorteilhaft, einen zylindrischen oder nahezu zylindrischen Anfangsabschnitt 21 über eine Höhe mindestens der Hälfte des Nenndurchmessers der zu extrahierenden Schraube zu haben, da diese Höhe als Abschnitt eine zentrierende Führung des abgespanten Materials 54 zum Verdichtungsbereich 55 gestattet und eine entsprechende Längsführung bietet. Gleichzeitig ist mit ihm eine gewisse Fixierungslänge des zu entnehmenden Schaftabschnittes 51 zu erreichen.

Die Fig. 6 zeigt ein weiteres Ausführungsbeispiel der Erfindung, bei dem sich an den zylindrischen Innenwandabschnitt 21 in Höhe des Schaftüberganges 323 eine Abfolge von Stufen 322 anschliesst, deren Durchmesser jeweils zylindrisch ist, wobei jede Stufe gegenüber einer proximal angeordneten Stufe aber jeweils einen etwas kleineren Durchmesser aufweist, so dass sich gegenüber der hintersten, distalen Stufe 324 ein verkleinerter Innendurchmesser ergibt. Die Stufen können alle die gleiche Grösse aufweisen. Sie können auch unterschiedlich lang sein, womit sich bei gleicher Stufenhöhe von beispielsweise 0,1 mm ein im umhüllenden Winkel variabler Konus ergibt.

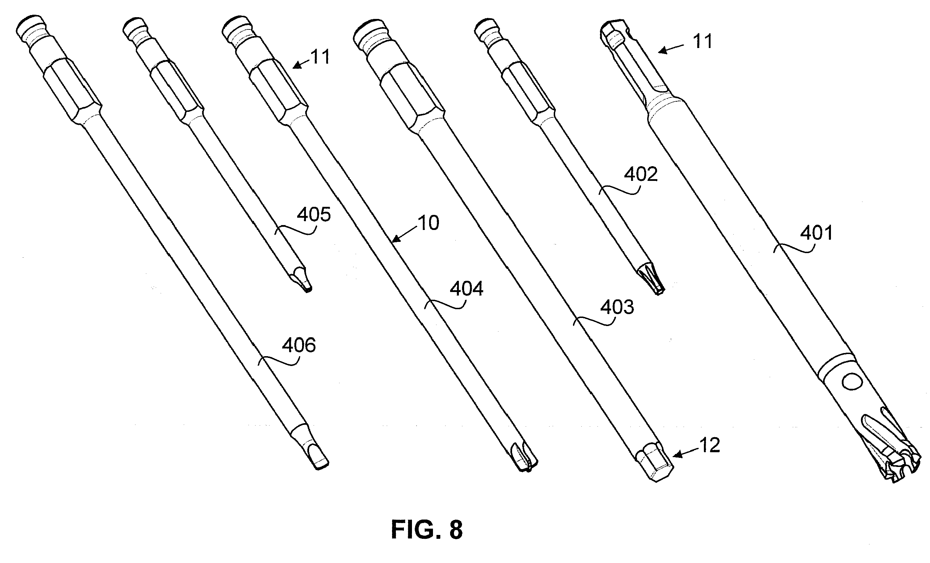

Die Fig. 8 zeigt schliesslich eine perspektivische Ansicht von 6 Werkzeugspitzen 401, 402,403, 404, 405, und 406 in einer Übersicht. Alle Werkzeugspitzen verfügen über einen Verbindungsadapter 11 zum Einsatz in einem Handgriff, sowie eine Bearbeitungsspitze 12. Bei der Bearbeitungsspitze 12 kann es sich um eine Hohlfräse, einen Torxkopf, einen Inbuskopf, einen Torqkopf, einen Phillips-Kopf oder einen normalen Schlitzschraubendreherkopf handeln. Es ist von Vorteil, an Stelle von für Implantate verwendete Metalle zum mehrfachen Einsatz beispielsweise einen HSS-Stahl oder einen Federstahl für Einwegwerkzeuge vorzuschlagen; insbesondere also aus nicht korrosionsbeständigen Stählen. Hier ist es dann wesentlich, gerade im Umfeld von aggressiven Körperflüssigkeiten, sicherzustellen, dass diese Werkzeuge nur einmal verwendet werden. Hiefür können irisierende Plasmabeschichtungen beispielsweise gemäss "Neuartige dekorative Farbschichten durch Plasma-Beschichtung" von Volker Bucher aus "Vakuum in Forschung und Praxis"; 18 (2006); Nr. 2; S.27-34, eingesetzt werden. Sie weisen den Vorteil auf, dass sie durch unterschiedliche Schichtdicken unterschiedliche irisierende Farben darstellen, sodass verschiedene Grössenordnungen von Werkzeugen in Sortimenten entsprechend zusammengestellt werden können. Solche Beschichtungen sind gesundheitlich unbedenklich, da sie für Implantate zugelassen sind. Wenn dementsprechend bei einem Einsatz eines Werkzeuges wie beispielsweise den Bearbeitungsspitzen der Werkzeuge 401 bis 406 entsprechende Beschichtungsanteile im Körper verbleiben ist dies unbedenklich, abgesehen davon, dass die Mengen aufgrund der wenige Atomschichten umfassenden Beschichtung infinitesimal sind. Beim Stahl kann es sich um Chrom-Vanadium Federstahl auf Siliziumbasis handeln, wobei dieser in der Analyse neben Fe aus 0,63 bis 0,73 % C, 1,5 bis 2,5 % Si, 0,2 bis 1 % Cr, 0,2 bis 1 % Mn und 01 bis 0,3 % V besteht. Die Prozentangaben sind Gewichtsprozente.

Der wesentliche Vorteil der Plasmabeschichtung liegt darin, dass es möglich ist, gegenüber den nicht korrosionsbeständigen Stählen ein temporär korrosionsbeständiges Material auszuwählen. Unter kurzzeitig korrosionsbeständig versteht man eine Plasmabeschichtung eines Werkzeugs dann, wenn das Werkzeug nach dem Auspacken aus einer beispielsweise sterilen Verpackung bis nach dem einmaligen Einsatz nicht korrodiert. Ein einmaliger Einsatz kann dabei zwischen Minuten und mehreren Stunden bis maximal unter einen Tag dauern. Obwohl dieses Material härter sein kann und zumeist ist, ergeben sich beim Einsatz und durch die Reinigung nach dem Einsatz an den Werkzeugen, sowohl an der Bearbeitungsspitze 12, als auch am Schaft 10, sofort Konosionsspuren, die optisch als Fehler in der Oberfläche erkennbar sind. Wenn also bei der Nachbereitung einer Operation nicht bereits die Werkzeuge entsorgt werden, so ist jedenfalls nach einem Sterilisationsschritt sofort erkennbar, dass es sich um gebrauchte Gegenstände handelt, aufgrund der optischen Veränderung. in den irisierenden Oberflächen.

### BEZUGSZEICHENLISTE

- 10: Schaft
- 11: Verbindungsadapter
- 12: Werkzeugkopf
- 13: Schaftübergang
- 14: Querloch
- 15: Fräszahn
- 16: Spanabführnut
- 17: Innenhohlraum
- 18: verdickte Frässpitze
- 19: Schaftübergang
- 21: zylindrischer Innenwandabschnitt
- 22: konischer Innenwandabschnitt
- 23: Abschnittsübergang
- 31: Längsachse
- 32: Querlochachse
- 41: zylindrischer Hohlraumabschnitt
- 42: konischer Hohlraumabschnitt
- 51: Schraubenschaft
- 52: Knochenmaterial
- 53: gefrästes Bohrloch
- 54: Knochenmaterialanteil
- 55: komprimiertes Material
- 122: konischer Innenwandabschnitt
- 123: Abschnittsübergang
- 222: gekrümmter Innenwandabschnitt
- 223: Abschnittsübergang
- 322: stufenförmiger Innenwandabschnitt
- 323: Abschnittsübergang
- 324: kleiner Innendurchmesser
- 401-406: Werkzeuge

## Patentansprüche

1. Extraktionswerkzeug für das Entfernen von Knochenschrauben, insbesondere in der Orthopädie oder der Unfallchirurgie, bei der ein Hohlfräser (12) über das zu extrahierende Werkstück (51) einsetzbar ist, um einen dieses umgebenden Kanal (53) zu bohren, wobei der Hohlfräser (12) an seinem proximalen Ende über spanabhebende Elemente (15) und in seinem hohlen, zentrischen Innenraum über Eingriffselemente verfügt, so dass das Werkzeug nach einer vorbestimmbaren Vordringtiefe mit dem zu extrahierenden Werkstück (51) so fest verbunden ist, dass dieses extrahierbar ist, **dadurch gekennzeichnet, dass** die hohle Innenseite (21, 22) des Werkzeuges über einen mindestens teilweise glatten, in seinem Durchmesser sich distal verjüngenden Innenwandabschnitt (42; 22; 122; 222; 322) verfügt.

2. Extraktionswerkzeug gemäss Anspruch 1, **dadurch gekennzeichnet, dass** die hohle Innenseite (21, 22) des Werkzeuges über einen glatten zylindrischen Innenwandabschnitt (41; 21) verfügt.

3. Extraktionswerkzeug gemäss Anspruch 2, **dadurch gekennzeichnet, dass** der glatte zylindrische Innenwandabschnitt (41; 21) eine Höhe aufweist, die zwischen der Hälfte bis zum Vierfachen seines Nenninnendurchmessers beträgt, vorteilhafterweise zwischen dem Ganzen und dem Doppelten seines Nenninnendurchmessers.

4. Extraktionswerkzeug gemäss Anspruch 1 oder 2, **dadurch gekennzeichnet, dass** der mindestens teilweise glatte, in seinem Durchmesser sich distal verjüngende Innenwandabschnitt (22; 122; 222; 322) eine Höhe aufweist, die zwischen der Hälfte bis zum Vierfachen der Höhe des glatten zylindrischen Innenwandabschnitts (41; 21) beträgt, vorteilhafterweise zwischen dem Ganzen und dem Doppelten der besagten Höhe.

5. Extraktionswerkzeug gemäss einem der Ansprüche 1 bis 4, **dadurch gekennzeichnet, dass** der mindestens teilweise glatte, in seinem Durchmesser sich distal verjüngende Innenwandabschnitt (22; 122) einen Hohlkonus mit konstantem Winkel gegenüber der Längsachse des Hohlraums (17) aufspannt.

6. Extraktionswerkzeug gemäss Anspruch 5, **dadurch gekennzeichnet, dass** der Winkel gegenüber der Längsachse des Hohlraums (17) zwischen 1 und 10 Grad, vorteilhafterweise zwischen 2 und 7 Grad und insbesondere zwischen 3 und 6 Grad ist.

7. Extraktionswerkzeug gemäss einem der Ansprüche 1 bis 4, **dadurch gekennzeichnet, dass** der in seinem Durchmesser sich distal verjüngende Innenwandabschnitt (222) einen gekrümmten Hohlkonus mit einem sich von proximal zu distal im Wert ansteigenden Winkel bis 7 oder 10 Grad gegenüber der Längsachse des Hohlraums (17) aufspannt.

8. Extraktionswerkzeug gemäss Anspruch 7, **dadurch gekennzeichnet, dass** der in seinem Wert ansteigende Winkel von 0 Grad oder in einem Bereich zwischen 0 und 2 Grad ansteigt.

9. Extraktionswerkzeug gemäss einem der Ansprüche 1 bis 4, **dadurch gekennzeichnet, dass** der Innenwandabschnitt (322) aus einer Abfolge von sich von proximal zu distal im Durchmesser verkleinernden zylindrischen Stufen besteht.

10. Extraktionswerkzeug gemäss einem der Ansprüche 1 bis 9, **dadurch gekennzeichnet, dass** der Hohlraum (17) in einem das Werkzeug mindestens einseitig öffnenden Querloch (14) mündet.

11. Werkzeug gemäss einem der Ansprüche 1 bis 10; **dadurch gekennzeichnet, dass** das Werkzeug über einen Kernkörper aus einem Stahl vorbestimmter Härte besteht und dass das Werkzeug mindestens im Bereich der Werkzeugenden (12) eine Beschichtung aufweist, die aus einer kurzzeitig korrosionsbeständigen Plasmabeschichtung besteht.

12. Werkzeug gemäss Anspruch 11, **dadurch gekennzeichnet, dass** der Stahl ein Chrom-Vanadium Federstahl auf Siliziumbasis ist, insbesondere ein Stahl, der neben Eisen aus 0,63 bis 0,73 % Kohlenstoff, 1,5 bis 2,5 % Silizium, 0,2 bis 1 % Chrom, 0,2 bis 1 % Mangan und 01 bis 0,3 % Vanadium besteht, wobei die Prozentangaben Gewichtsprozente sind.
